# EUROPEAN PATENT APPLICATION

(11) **EP 2 289 584 A1**
(43) Date of publication of application: **02.03.2011**
(21) Application number: 10012527.7
(22) Date of filing: 06.12.2002
(51) Int. Cl.: A61M 16/01, A61M 5/00

(54) **CO2 monitored drug infusion system**

(30) Priority: 06.12.2001 US 337220 P
(62) Divisional of application: 05024579.4
(71) Applicant: CareFusion 303, Inc., San Diego, CA 92130 (US)
(72) Inventor: Vanderveen, Timothy, W., Poway CA 92064 (US)
(74) Representative: Richards, John

(57) **Abstract**

A patient care system (90) comprising an interface unit (100), an infusion pump unit (150a), and a capnography unit (150b) is disclosed. The infusion pump unit (150a) is used to administer medical fluids to a patient, including the administration of anesthethics, analgesics, or sedatives. The capnography unit (150b) provides monitoring of the patient's expired air, in particular, the end tidal CO₂ levels in the expired air and the respiration rate. When the capnography unit (150b) indicates to the interface unit that the end tidal CO₂ level and/or pulse rate has reached a point outside a specified range, the interface unit (100) initiates visual and audio alarms and controls the infusion pump unit (150a) by modifying the flow or by shutting off the pump unit. In patient controlled analgesia applications, the interface unit (100) may block patient-controlled bolus doses of the analgesic until reset by a qualified medical professional or until the measured capnography values return to a normal range. The interface unit (100) also contains communication ports, which the interface unit can use to send signals to external devices, such as to alert medical personnel.

## Description

### BACKGROUND

The present invention relates generally to a patient care system in which medical fluid is administered to a patient while the patient is monitored for a physical condition, and more particularly, to a system and method in which medical fluid is administered to a patient while the expired air of the patient is monitored for a specific component.

Programmable infusion systems are commonly used in the medical field to deliver a wide range of drugs and fluids to patients in a variety of settings. For example, syringe pumps, large volume pumps (herein "LVP"), and flow controllers are used in hospitals, clinics, and other clinical settings to deliver medical fluids such as parenteral fluids, antibiotics, chemotherapy agents, anesthetics, analgesics, sedatives, or other drugs. Single or multichannel systems are available, and different systems have various levels of sophistication, including automatic drug calculators, drug libraries, and complex delivery protocols. Still another type of drug delivery system is a patient controlled analgesia (herein "PCA") pump. With a PCA pump, the patient controls the administration of the narcotic analgesics since the patient is usually in the best position to determine the need for additional pain control. PCA is commonly administered via a stand-alone type of infusion device dedicated solely for PCA use. Examples of PCA devices are disclosed in U.S. Patent No. 5,069,668 to Boydman and U.S. Patent No. 5,232,448 to Zdeb.

Regardless of the type of pump system used, a serious side effect of the administration of drugs, particularly anesthetics, analgesics or sedatives, can be central nervous system and respiratory depression which can result in serious brain damage or death. For example, the infusion of anesthetics, analgesics or sedatives using a syringe pump or LVP requires careful supervision by a trained medical professional to avoid overdosing. Even with infusion systems having sophisticated automatic programming and calculation features designed to minimize medication errors, it is not uncommon for patients to experience respiratory depression or other deleterious effects during the administration of narcotic analgesics or sedatives during in-patient or out-patient clinical procedures. Even in PCA applications, where overdoses are typically prevented by the patient falling asleep and therefore being unable to actuate a delivery button, there have been cases of respiratory and central nervous system depression and even death associated with the administration of PCA. The causes include clinical errors in programming the PCA device, errors in mixing or labeling analgesics, device malfunction, and even overzealous relatives who administer extra doses of analgesics by pressing the dose request cord for the patient.

Because of the potential dangers of narcotic analgesic overdose, narcotic antagonists such as naloxone (Narcan^{tm}) are widely available and commonly used in hospitals for reversal of respiratory and central nervous system depression. However, the effectiveness of such narcotic antagonists is highly dependent on prompt recognition and treatment of respiratory and central nervous system depression, as such depression can cause brain damage or even death due to lack of oxygen. Thus, respiratory and central nervous system depression must be recognized and treated promptly to assure a higher probability of successful recovery. Therefore, it would be desirable to monitor the actual physical condition of the patient to find respiratory or nervous system depression so that immediate remedial measures may be taken.

For the detection of potential respiratory depression associated with the administration of narcotic analgesics, sedatives, or anesthetics, a system that indicates a patient's respiratory status and cardiac status without the need to invasively measure or sample the patient's blood is particularly desirable and useful. Non-invasive pulse oximetry is one such method used to monitor the oxygen saturation of a patient's blood and the patient's pulse rate. The combination of the blood oxygen saturation and pulse rate can be an important indicator of overall patient respiratory and cardiac status.

One common approach to non-invasive pulse oximetry uses a dual-wavelength sensor placed across a section of venous tissue such as the patient's digit to measure the percentage of hemoglobin oxygenated in the arterial blood, and thereby measures the patient's oxygen saturation level. In addition, since the oxygenated hemoglobin at a specific tissue position is pulsatile in nature and synchronous with the overall circulatory system, the system indirectly measures the patient's pulse rate. Examples of pulse-oximetry sensors are disclosed in U.S. Patent No. 5,437,275 to Amundsen et al. and U.S. Patent No. 5,431,159 to Baker et al.

U.S. Patent No. 5,957,885 to Bollish et al. ("Bollish"), which is incorporated herein in its entirety, discloses an infusion system utilizing an associated pulse oximetry monitor to measure the oxygen saturation level of a patient's blood and to block operation of the PCA pump if the measured SpO₂ and/or pulse rate values fall outside of a predetermined range. However, while pulse oximetry provides an indication of respiratory depression, the warning triggered by the pulse oximetry signal is derived from oxygen levels in the patient's blood, and therefrom may not be early enough to reverse the respiratory depression or prevent detrimental effects thereof.

Another means of monitoring the respiratory status of a patient is by measuring and charting the concentration of CO₂ in the patient's expired air, a procedure known as capnography. In particular, current capnography devices utilize spectroscopy, for example infrared, mass, Raman, or photo-acoustic spectroscopy, to measure the concentration of CO₂ in air flowing through a non-invasive nose and/or mouthpiece fitted to the patient (e.g., ORIDION Corporation, http://oridion.com; NOVAMETRIX Medical Systems Inc., http://www.novametrix.com, and U.S. Patent Application Publication US 2001/0031929 A1 to O'Toole). Capnographic CO₂ waveforms and indices such as end tidal CO₂ concentration (herein "ETCO₂"), or the concentration of CO₂ just prior to inhaling, are currently used to monitor the status of patients in operating rooms and intensive care settings. However, a capnography device has never been integrated into a drug delivery system to automatically provide an alarm, suspend delivery, or otherwise alter the course of drug delivery.

Hence, those skilled in the art have recognized a need for a patient care system and method that can monitor the physical condition of a patient through analysis of his or her expired air, and can control the infusion of medical fluids to the patient based on the analysis. Further, those skilled in the art have recognized a need for a patient care system and method that can monitor the expired air of a patient and provide an alarm or other indication to a care giver when an air component is outside a predetermined range or rate of change so that remedial action may be taken as soon as possible, if necessary. The present invention satisfies these needs and others.

### SUMMARY OF THE INVENTION

Briefly and in general terms, the present invention is directed to an apparatus and method for a patient care system comprising a pump for delivery of a medical fluid to a patient, a controller in communication with the pump for controlling operation of the pump, a monitor unit that monitors the expiration air of the patient and provides a measured value of a selected component of the expiration air to the controller, and a memory with which the controller is connected, the memory comprising a stored range of acceptable values of the selected component of expiration air, wherein the controller compares the measured value of the selected component received from the monitor unit to the range of acceptable values for the component stored in the memory and if the measured value is outside the range stored in the memory, the controller performs a predetermined action.

In another aspect, the monitor unit monitors the expiration air of the patient for CO₂ and provides a measured value of the CO₂ to the controller. The controller automatically adjusts the rate of delivery of the medical fluid in accordance with the CO₂ in the patient's expired air, and in a more detailed aspect, the controller automatically suspends delivery of the medical fluid by the pump to the patient if the measured value of the CO₂ in the expired air of the patient is outside the stored range of acceptable values.

In other aspects in accordance with the invention, the patient care system further comprises a PCA dose request switch connected to the controller with which the patient may request the pump to infuse a quantity of analgesic, wherein prior to allowing the pump to infuse the quantity of analgesic, the controller compares the measured value of the CO₂ received from the monitor unit to the range of acceptable values for CO₂ stored in the memory and if the measured value is outside the range stored in the memory, the controller does not permit the pump to infuse the requested quantity of analgesic to the patient. In another aspect, a PCA dose request switch is connected to the controller with which the patient may request the pump to infuse a quantity of analgesic, wherein prior to allowing the pump to infuse the quantity of analgesic, the controller compares the rate of change of the CO₂ received from the monitor unit to the range of acceptable values for CO₂ stored in the memory and does not permit the pump to infuse the requested quantity of analgesic to the patient if the rate of change is not consistent with the acceptable values.

In more detailed aspects, the patient care system further comprises a display on which is displayed a CO₂ waveform of the patient as derived from a series of measured CO₂ values provided by the monitor unit. Further, the monitor unit monitors the expiration air of the patient for end tidal CO₂ and provides a measured value of the end tidal CO₂ to the controller. The controller automatically adjusts the rate of delivery of the medical fluid in accordance with the end tidal CO₂ in the patient's expired air. In another aspect, the controller automatically suspends delivery of the medical fluid by the pump to the patient if the measured value of the end tidal CO₂ in the expired air of the patient is outside the stored range of acceptable values.

In yet further detail, the memory in which the range of acceptable values of the selected component is stored is located at a position removed from the pump. In another aspect, the memory in which the range of acceptable values of the selected component is stored is located in the pump.

In yet further aspects, the patient care system comprises an oximetry unit connected to the controller that monitors the blood of the patient and provides a measured value of the oxygen saturation of the patient's blood to the controller, wherein the memory comprises a stored range of acceptable values of the oxygen saturation of blood, wherein the controller compares the measured value of the oxygen saturation received from the oximetry unit to the range of acceptable values for the oxygen saturation stored in the memory and if the measured value is outside the range stored in the memory, the controller performs a predetermined action. In further detail, the controller automatically adjusts the rate of delivery of the medical fluid in accordance with either of the CO₂ in the patient's expired air or the oxygen saturation of the patient's blood. In yet even further aspects, the oximetry unit also monitors the pulse rate of the patient and provides a measured value of the pulse rate to the controller, wherein the memory comprises a stored range of acceptable values of the pulse rate, wherein the controller compares the measured value of the pulse rate received from the oximetry unit to the range of acceptable values for the pulse rate stored in the memory and if the measured value is outside the range stored in the memory, the controller performs a predetermined action. Further, the controller automatically adjusts the rate of delivery of the medical fluid in accordance with any of the CO₂ in the patient's expired air, the oxygen saturation of the patient's blood, or the patient's pulse rate.

In accordance with method aspects, there is provided a method for controlling patient self-administration of fluid infusions comprising monitoring patient conditions by connecting a capnography unit to the patient, wherein the capnography unit is capable of monitoring the concentration of carbon dioxide in the expired air of a patient, connecting the capnography unit to an interface unit including a microprocessor and a user interface adapted to provide an interface with a user, inputting patient condition limits into the interface unit, comparing monitored patient conditions to patient condition limits in said interface and generating a signal indicative of said comparison, connecting the patient to an infusion unit, wherein the infusion unit communicates with the interface unit, said infusion unit is capable of performing fluid infusion to the patient in accordance with infusion unit specific information. requesting fluid infusions from the infusion unit by patient activation of the infusion unit, performing fluid infusion with the infusion unit in accordance with a predetermined infusion protocol in response to said patient activation and in response to a signal from the interface unit representative of the monitored patient conditions being within said patient condition limits, and disabling the infusion unit and terminating the fluid infusion in response to a signal from the interface unit representative of the monitored patient conditions being outside the patient condition limits.

In yet further apparatus aspects, there is provided a drug infusion pump for use with a container containing a given drug, said container including a machine readable label, the label specifying an identifier of the given drug and possibly other information about the given drug, said pump comprising a pump mechanism which during operation causes the given drug to be delivered to a patient from the container, a programmable controller controlling the pump mechanism, a monitor unit that monitors the expiration air of a patient to measure a selected component of that air, and that provides a measured value representative of the measured component, a memory storing a drug library, said drug library containing a plurality of drug entries, there being associated with each drug entry a set of associated drug delivery parameters for configuring the drug infusion pump, the memory also storing the selected component of the patient's expiration air, there being associated with the selected component a range of acceptable values, a label reader which during use reads the contents of the label on the container, and means responsive to the label reader for identifying an entry in the drug library that corresponds to the given drug and configuring the programmable controller by using the set of drug delivery parameters associated with the identified entry from the drug library, wherein the programmable controller is configured to receive the measured value, compare the measured value to the range of acceptable values of the selected component, and to control the pump mechanism in accordance with the comparison.

Other features and advantages of the present invention will become more apparent from the following detailed description of the invention when taken in conjunction with the accompanying drawings.

### BRIEF DESCRIPTION OF THE DRAWINGS

FIG. 1 is a front view of an embodiment of a patient care system according to aspects of the present invention showing a large volume pump unit, a CO₂ monitoring unit, and a central interface unit interconnecting the large volume pump unit and the CO₂ monitoring unit;
FIG. 2 is a front view of a patient care system according to another preferred embodiment of the present invention showing a patient controlled analgesia unit, a CO₂ monitoring unit, and a central interface unit interconnecting the large volume pump unit and the CO₂ monitoring unit;
FIG. 3 is a back view of a central interface unit of the patient care system of FIGS. 1 and 2;
FIG. 4 is a block diagram of a central interface unit of the patient care system of FIG. 2;
FIG. 5 depicts an information display of the central interface unit of FIG. 4 during setup of a CO₂ monitoring unit showing areas for the input of values;
FIG. 6 depicts another information display of the central interface unit of FIG. 4 during setup of the CO₂ monitoring unit with values entered;
FIG. 7 depicts another information display of the central interface unit of FIG. 4 during setup of a PCA unit showing the required selection of units;
FIG. 8 depicts another information display of the central interface unit of FIG. 4 during setup of the PCA unit showing the unit selections made;
FIG. 9 depicts another information display of the central interface unit of FIG. 4 during setup of the PCA unit showing values entered;
FIG. 10 depicts an information display of the central interface unit of FIG. 4 after completion of setup and during operation;
FIG. 11 depicts an information display of the central interface unit of FIG. 4 with the patient care system in an alarm mode;
FIG. 12 is a front view of another embodiment of a patient care system in accordance with aspects of the present invention having a PCA pump unit, a CO₂ monitor unit, and a pulse oximeter monitor unit;
FIG. 13 is a front view of another embodiment of the patient care system in accordance with aspects of the present invention having a PCA pump unit and a combined CO₂/pulse oximeter monitor unit both of which are mounted to a central interface unit;
FIG. 14 depicts an information display of the central interface unit of FIG. 13 during setup of the CO₂/pulse oximetry unit showing value fields;
FIG. 15 depicts another information display of the central interface unit of FIG. 13 during setup of the CO₂/pulse oximetry unit showing values entered in the fields to establish ranges of acceptable values of physiological parameters; and
FIG. 16 is a block diagram of an infusion pump according to aspects of the present invention including an integrated CO₂ monitor and a pulse oximeter.

### DETAILED DESCRIPTION OF PREFERRED EMBODIMENTS

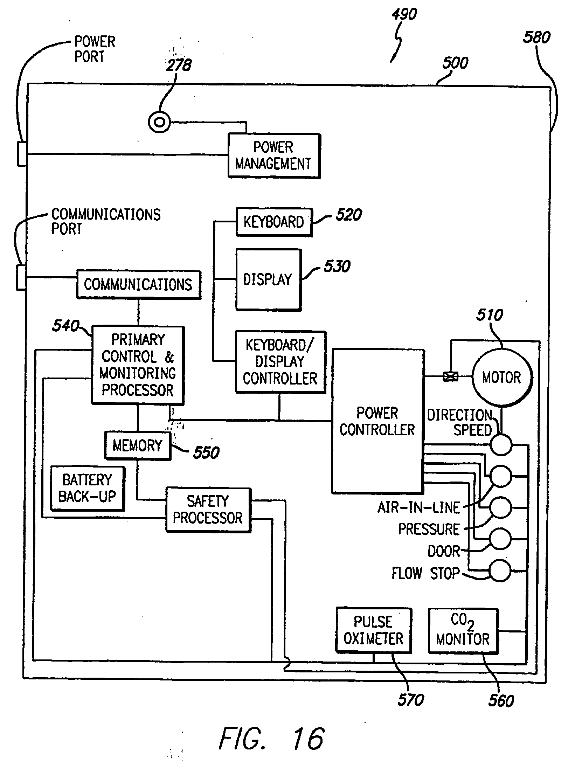

The following preferred embodiments of the present invention are described generally in the context of the programmable modular patient care systems disclosed in U.S. Patent No. 5,713,856 filed March 13, 1995 entitled Modular Patient Care System, and U.S. Patent No. 5,957,885 filed November 6, 1996 entitled Oximetry Monitored, Patient Controlled Analgesia System. Both of these patents are owned by the assignee of the present application, and are incorporated herein in their entirety by reference. However, a person skilled in the art will recognize that the disclosed methods and apparatus are readily adaptable for broader application, including but not limited to other patient care systems and drug infusion pump systems. Moreover, as will also be appreciated by persons of ordinary skill in the art, a CO₂ monitored drug delivery system according to the present invention can also be provided as a stand alone integral unit, as discussed in detail below in connection with FIG. 16.

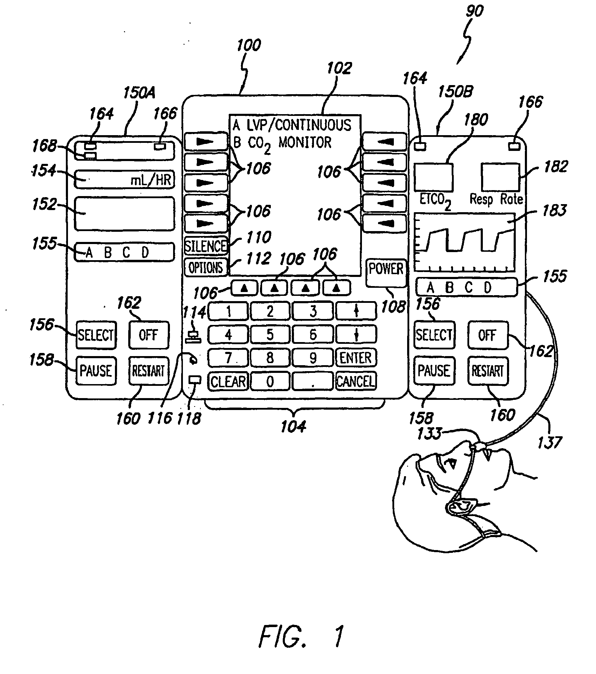

Referring now to the drawings with more particularity, in which like reference numerals among the several views indicate like or corresponding elements, FIG. 1 shows a front view of a modular, programmable patient care system 90 according to a preferred embodiment of the present invention. The patient care system 90 comprises a central interface unit 100, a pump unit 150A, a unit that monitors a patient's expired air 150B to determine the concentration of a selected component, such as a capnography unit 150B to measure CO₂ (also termed "CO₂ monitoring unit"), and an expired air sampling device 133. Although not shown, both the pump unit 150A and the CO₂ unit 150B are connected to the patient. Although FIG. 1 shows only two functional units, i.e., the pump unit 150A and the CO₂ monitoring unit 150B, attached to the central interface unit 100, the patient care system 90 may additionally comprise other functional units, depending on a patient's particular needs. For example, one or more additional functional units can be connected to either the pump unit 150A or the capnography unit 150B, including but not limited to large volume pumps, flow controllers, syringe pumps, PCA pumps, CO₂ monitors, other air analysis monitors, pulse oximetry monitors, electrocardiographs, invasive and noninvasive blood pressure monitors, auditory evoked potential (AEP) monitors for monitoring the level of consciousness, cerebral blood flow monitors or cerebral oxygenation monitors, and others.

The central interface unit 100 generally performs five functions in the patient care system 90:
(1) it provides a physical attachment of the patient care system 90 to structures such as IV poles and bed rails;
(2) it provides a power supply to the patient care system 10;
(3) it provides an interface between the patient care system 10 and external devices;
(4) except for certain specific information, it provides a user interface with the patient care system 90; and
(5) it monitors and controls the overall operation of the patient care system 90, including the integration of signals from monitor modules and/or pump modules in order to signal alerts and/or affect operation of one or more pump modules.

The central interface unit 100 contains an information display 102 that may be used during setup and operating procedures to facilitate data entry and editing. The information display 102 may also display various operating parameters during operation such as the drug name, dose, infusion rate, infusion protocol information, patient lockout interval for PCA applications, ETCO₂ and pulse rate limits for the capnography unit 150B. If other functional units are attached, such as a pulse oximeter, the information display 102 can display oxygen saturation, pulse rate limits, and/or other functional unit-specific information. The information display 102 is also used to display instructions, prompts, advisories, and alarm conditions to the user.

The central interface unit 100 also contains a plurality of hardkeys 104 for entering numerical data and, along with softkeys 106, for entering operational commands. In addition, the central interface unit 100 further contains a POWER ON hardkey 108 for turning electrical power on or off to the central interface unit 100, a SILENCE hardkey 110 for the temporary disablement of the audio functionality of the central interface unit 100, and an OPTIONS hardkey 112 for allowing user access to available system or functional unit options. The central interface unit 100 may further contain an external computer indicator 114 for indicating that the patient care system 90 is communicating with a compatible external computer system, an external power indicator 116 to indicate that the central interface unit 100 is connected to and operating with an external power source, and an internal power indicator 118 to indicate that the central interface unit 100 is operating with the use of an internal power source. The central interface unit 100 may also include a tamper-resistant control function (not shown) which can lock out a predetermined set of controls.

The pump unit 150A and the capnography unit 150B each include a channel position indicator 155 that illuminates one of the letters "A", "B", "C", or "D" to identify the channel position of that functional unit with respect to the patient care system 90. For example, the patient care system 90 contains two channel positions A and B, with A to the immediate left of the central interface unit 100 (such as the pump unit 150A of FIG. 1), and B to the immediate right of the central interface unit 100 (such as the capnography unit 150B of FIG. 1). Because both the pump unit 150A in channel A and the capnography unit 150B in channel B are attached, as shown in FIG. 1, the information display 102 on the interface unit 100 indicates A and B (note: in this embodiment, the pump unit 150A is designated on the information display 102 as "LVP/Continuous" and the capnography unit 150B is designated on the information display 102 as "CO₂ MONITOR"). When the desired functional unit is selected by depressing the CHANNEL SELECT key 156 of a corresponding functional unit, the information display 102 is configured so as to act as the user interface for the selected functional unit. Specifically, the information display 102 is configured in accordance with a function specific domain to provide function specific displays and softkeys, as will become clear from the description of an example below.

Each functional unit has a CHANNEL SELECT key 156 for selection of the functional unit, a PAUSE key 158 (1) for pausing an infusion if the functional unit is a pump and if infusion is occurring or (2) for pausing a monitoring function if the functional unit is a monitoring unit, a RESTART key 160 for resuming a previously paused infusion or monitoring function, and a CHANNEL OFF key 162 for deselecting the channel, and, if the functional unit on the channel was the only functional unit operating, for powering off the patient care system 90. In addition, the pump unit 150A and the capnography unit 150B each contain an ALARM indicator 164 to indicate an alarm condition and a STANDBY indicator 166 to indicate a standby condition. The pump unit 150A additionally contains an INFUSING indicator 168 to indicate an infusing condition. Each indicator illustratively illuminates when the respective functional unit is in the respective condition.

The pump unit 150A contains a channel message display 152, that may be used to display informational, advisory, alarm, or malfunction messages, and a rate display 154 which may be used to display, for example, the infusion rate at which the pump unit is operating. The pump unit 150A may also include a door lock (not shown) for providing security for enclosed narcotics or other medications to be infused. As known in the prior art, the pump unit 150A can be either a PCA pump, a syringe-based pumping system, an LVP, a parenteral type, or other appropriate configurations as can be readily determined by one skilled in the art. The pump unit 150A includes standard pumping and safety mechanisms to control various functions performed by the pumping device such as control of fluid delivery to the patient and monitoring of the fluid path for occlusion or air-in-line.

Connected to the capnography unit 150B is an expired air sampling device 133 which preferably collects expired air from the patient's nose and mouth and optionally supplies oxygen to the patient. The expired air travels to the capnography unit 150B through the line 137 where it is analyzed in real-time for CO₂ concentration by the capnography unit 150B, preferably using infrared spectroscopy analysis. However, other CO₂ analysis techniques may be used as discussed above and as understood by persons of ordinary skill in the art. Alternatively, the sampling device 133 can include a sensor (not shown) for directly analyzing the expired air and sending a signal via the connection 137 or via a wireless communication system (not shown) to the monitor unit 150B. The capnography unit 150B includes several displays 180, 182, and 183 for displaying data to the user. For example, the end tidal CO₂ (herein "ETCO₂") display 180 displays a numeric value for the concentration of CO₂ after expiration and before inhalation preferably in units of mm Hg or %. The respiration rate display 182 displays a rate value depicting the patient's current respiration rate, for example as determined by frequency analysis of the CO₂ waveforms. The waveform display 183 displays CO₂ concentration in the patient's blood over time. Data shown in the waveform display 183 preferably can be selectively extended or compressed for analysis of wave characteristics or for analysis of trends. The data shown in the displays 180, 182 and/or 183 may be smoothed, corrected, time averaged analyzed, or otherwise manipulated before display to provide optimal clinical value to the user. For example, the capnography unit 150B could perform a running average to smooth the CO₂ waveform, and the horizontal time axis may be paused and/or adjusted for either CO₂ wave analysis or trend analysis.

As will be discussed in more detail below, data generated by the capnography unit 150B is provided to the central interface unit 100, and may be used to trigger an alarm, to signal an advisory on the information display 102, to automatically stop operation of the pump unit 150A, or to otherwise adjust or control delivery of a drug or other medical fluid by the pump unit 150A. For example, the interface unit 100 could be programmed to automatically stop the pump 150A if the patient's ETCO₂ values fall outside a predetermined range of acceptable values. Alternatively, the pump 150A and the monitor 150B could communicate directly with each other to affect delivery of fluid to this patient based upon the monitored parameters. In yet another embodiment, the capnography monitor 150B or interface unit 100 includes a waveform analysis algorithm to analyze the capnography waveform and affect operation of the pump 150A based upon certain waveform characteristics as are known in the art. In still another embodiment of the present invention, the interface unit 100 includes a multi-parametric algorithm to calculate one or more indices of patient status using data from a number of different attached physiological monitors, and uses the calculated indices to affect control of the pump 150A.

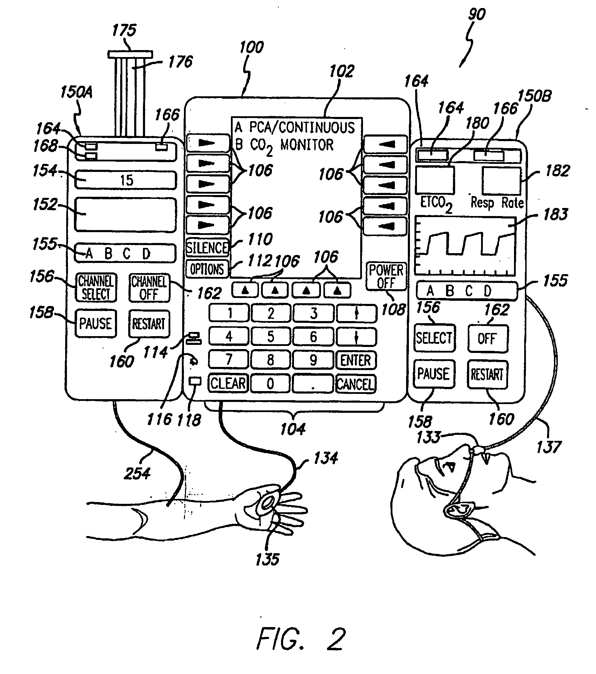

FIG. 2 shows an alternative embodiment of a patient care system 90, wherein the pump unit 150A is a PCA pump rather than an LVP pump. The pump unit 150A as shown has essentially the same interface displays and buttons as in FIG. 1; however, the pump unit 150A in FIG. 2 also includes a syringe pusher 175 and a syringe 176. The PCA pump unit 150A further includes an infusion pumping device within its housing that drives the syringe pusher 175 to infuse bolus doses of narcotic analgesics from the syringe to the patient in response to commands from the central interface unit 100. The display 154 display, for example, the infusion rate at which the PCA pump 150A is operating or the patient lockout interval. The interface unit 100, when configured with a PCA pump as the pump module 150A, includes a PCA patient dose request cord 134 connected to a handheld PCA dose request button 135 or other actuation device.

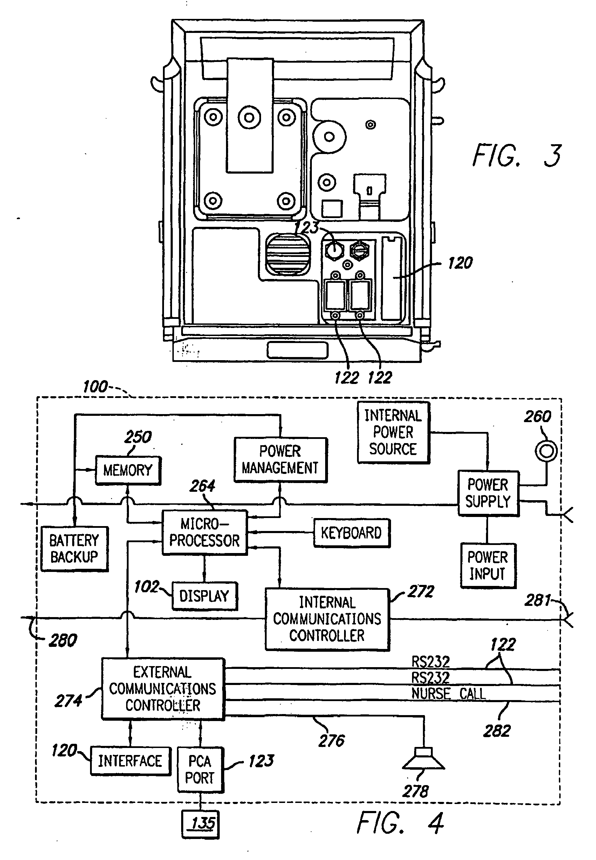

Referring now to FIG. 3, at the back of central interface unit 100 is at least one external communication interface 120, at least one interface port 122, and at least one PCA port 123. The external communication interface 120 and the interface port 122 may be used to download and upload information and data and may also act as an interface-to-patient monitoring network and nurse call system, or as an interface to external equipment such as a barcode reader to provide a means of inputting drug and/or patient information from medication or patient records or from information and identification devices, such as barcodes, located on the patient, the nurse or clinician, on the bag of medical fluid, and other devices. Performing these functions with the external communication interface 120 and the interface ports 122 provide greater functionality and adaptability, cost savings, and reduction in input error. In particular, clinical errors associated with programming the pump unit 150A would be reduced, thereby reducing the risks of respiratory depression associated with the administration of sedatives, narcotic analgesics, anesthetics, or other drugs from use of the pump unit 150A.

The PCA port 123 provides a connection between the central interface unit 100 and one end of the PCA patient dose request cord 134 (FIG. 2) if the pump unit 150A is a PCA pump. At an opposite end of the PCA patient dose request cord 134 is the hand-held dose request PCA button or other PCA actuation device 135, that can be actuated to request a dose of analgesic for the PCA patient. It is to be understood that although the central interface unit 100 contains a PCA port 123 in the preferred embodiment, the pump unit 150A may also contain a PCA port (not shown) that would provide a similar connection from the pump unit 150A, through a PCA patient dose request cord 134, to a dose request actuation device 135.

Referring now to FIG. 4, which depicts a block diagram of a central interface unit 100 in accordance with aspects of the present invention, a microprocessor controller 264 receives and processes data and commands from the user and communicates with the functional units and other external devices. The microprocessor controller 264 directly controls the external communication controller 274 which controls the PCA port 123 and the data flow through the interface ports 122 and/or external communication interface 120. The microprocessor controller 264 also controls the internal communications controller 272 which controls the internal communication ports 280 and 281. The internal communication ports 280 and 281 are included in each functional unit as well as the central interface unit 100 and provide data and command interfaces between the central interface unit 100 and the attached functional units 150A, 150B.

During operation of the patient care system 90 where the pump unit 150A is a PCA pump as shown in FIG. 2, when the dose request PCA actuation device 135 is actuated, the microprocessor 264 receives the dose request signal via the patient dose request cord 134 and the PCA port 123. If the microprocessor 264 determines that there are no limitations in administering a requested bolus dose of narcotic analgesics, the microprocessor 264 would then send a signal to the pump unit 150A, via the internal communications controller 272 and the internal communication port 280 and/or the port 281, instructing the pump unit 150A to administer the requested bolus dose.

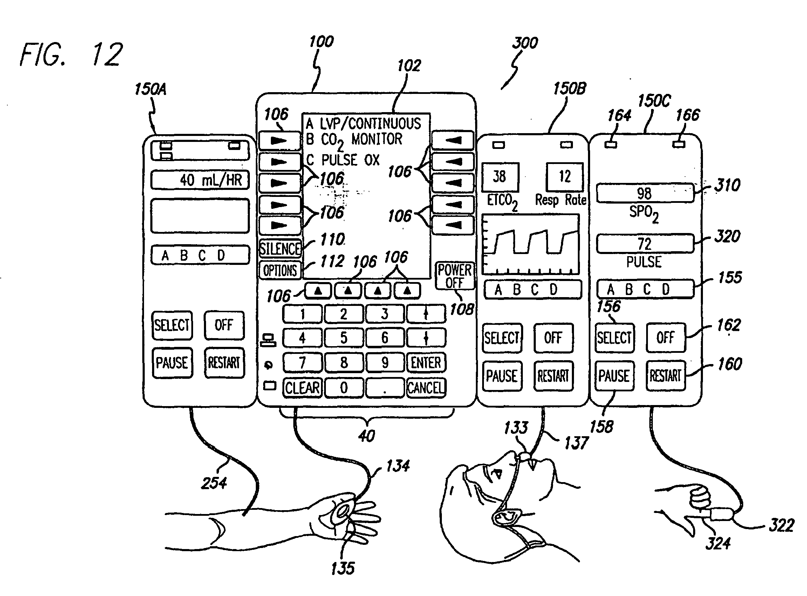

The microprocessor controller 264 also provides for the coordination of activities between the functional units, such as the pump unit 150A and the capnography unit 150B. For example, a clinician may set up the patient care system 90 with the pump unit 150A to provide PCA administration and the capnography unit 150B to monitor the ETCO₂ and the respiration rate of a PCA patient. Optionally, one or more additional monitors, such as a pulse oximetry unit 150C as shown in FIG. 12, may be serially attached to the patient care system 90 and set up to monitor blood oxygen saturation and pulse rate, for example, as described in more detail below. The clinician may specify a minimum and/or maximum value for ETCO₂, respiration rate, and/or other monitored parameters which thereby effectively sets a range of acceptable values for those parameters. If the patient's blood oxygen saturation or pulse rate is outside the selected acceptable range, such as in the case where it becomes less than the minimum or greater than the maximum levels set by the clinician, the ETCO₂ monitor 150B would send a trigger signal to the microprocessor controller 264 via the internal communications controller 272 and the internal communication port 280 and/or the port 281. In response, the microprocessor controller 264 may activate an audio alarm 276 to a speaker 278 as an example, send a visual alarm to the information display 102 (FIGS. 1 and 2), suspend operation of the pump unit 150A, adjust the flow rate of the pump unit 150A, and/or perform another predetermined function. For example, in response to an out-of-range ETCO₂ measurement in a PCA patient, the microprocessor controller 264 could cease all further administration of analgesics until after the exceedingly low or high ETCO₂ value and/or respiration rate situation are resolved, such as by clinician intervention. Alternatively, the microprocessor controller 264 may simply lock-out the PCA actuation device 135 so that the patient cannot obtain further self-administrations. Thus, after appropriate values have been set up, the central interface unit 100 provides communication and coordination between the pump unit 150A and the capnography unit 150B to ensure greater safety and decreased risk of injuries from respiratory depression.

In an alternative embodiment, rather than the microprocessor controller 264 suspending operation of the pump unit 150A in response to only an out-of-range signal from the capnography unit 150B or from another functional module, the microprocessor controller 264 would include program instructions for monitoring the changes in the CO₂ concentration data or other data generated by the capnography unit 150B and to make decisions on whether to interfere with the patient's control of the pump module 150A based upon the changes in the monitored data.

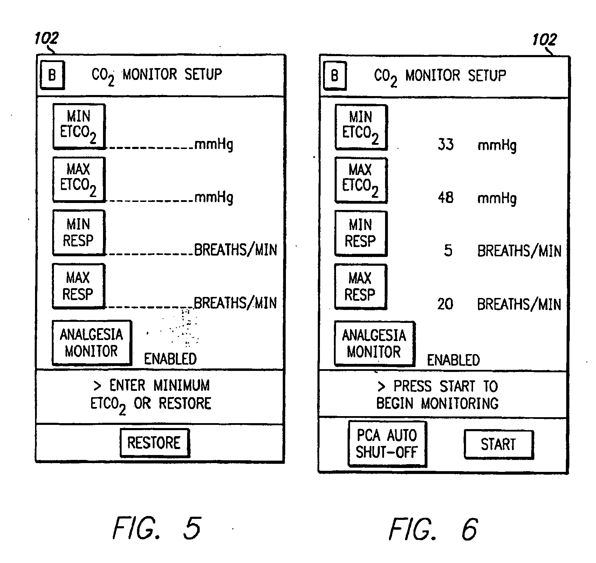

The interactions and functions of the central interface unit 100, the pump unit 150A, and the capnography unit 150B will now be described in conjunction with FIGS. 5-11 that show some of the step-by-step states of information display during the setup and operation of the patient care system 90. While the following example describes the setup of an operation of system 100 in a PCA setting utilizing a single PCA pump 150A and a single capnography monitor 150B, one skilled in the art will appreciate that the present invention encompasses programmed infusion protocols utilizing other types and numbers of infusion pumps and monitors.

To set up a preferred embodiment of the patient care system 90, the clinician first attaches the expired air sampling device 133 to the patient as shown in FIGS. 1 and 2. The clinician then selects the capnography unit 150B and its corresponding channel by pressing the SELECT key 156 on the capnography unit 150B. By selecting the capnography unit 150B, the information display 102 is configured so as to act as the user interface and thus provides capnography function specific displays and softkeys, as shown in FIG. 5. The clinician can either input the minimum and maximum values by pressing the respective softkey and entering the associated limit numbers or by restoring the previous values for the ETCO₂, and respiration rate by pressing the softkey below the RESTORE label.

FIG. 6 shows the information display 102 after the clinician has entered or restored previous values. Prior to starting capnography monitoring by pressing the softkey associated with the START label, the clinician may select the PCA auto shut-off option for one or more other functional units, such as the PCA unit 150A, so that the central interface unit 100 will shut off the selected functional unit(s) if the patient's ETCO₂, or respiration rate, or some combination thereof, falls outside of the specified maximum and minimum levels. Alternatively, the information display 102 could include parameters or selectable protocols for analyzing the patient's capnography waveform and setting limits on derived indices. Once capnography monitoring starts, the patient's ETCO₂, respiration rate, and capnography waveform are displayed in the displays 180, 181, and 182 of the CO₂ unit 150B, as previously described and shown in FIGS. 1 and 2. Although the preferred embodiment of the patient care system 90 automatically initiates both audio 276/278 and visual alarms 102 as well as notifies medical personnel, such as by triggering a nurse call 282, if the patient's ETCO₂ or respiration rate falls above or below specified maximum or minimum levels, the patient care system 90 can be configured such that the clinician can also select specific alarms and notifications to medical personnel in such an event.

In a preferred embodiment of the present invention, limit values for ETCO₂, respiration rate, and other parameters are stored in a data base in a memory 250 in the interface unit 100 (FIG. 4) or in the monitor 150B of the patient care system. Thus, rather than manually entering values using the numeric keys on the user interface 100 keypad 104 (FIG. 2), a user may recall pre-programmed values and/or configuration protocols from the stored data base to save time and minimize programming errors.

Storing a data base of institutional standards for drug infusion parameters and physiological parameter limits, such as the maximum and minium concentrations of CO₂ and the maximum and minimum values of respiration rate, also aids in standardizing the quality of care in a clinical setting. In some embodiments, infusion parameter values or physiological parameter limits may be entered automatically from a machine-readable label, for example by using a bar code reader (not shown) with the barcode label mounted on the bag or on the syringe or other medical fluid container in which the medical fluid to be infused is stored. Such infusion parameter values and physiological parameter values may also be entered by other means, such as through a connection with an external processor, such as a hospital server, through connection to a PDA, or other. Connections with these devices may be made in various ways, such as direct hardwired connection, infrared link, RF, use of an RF ID chip with RF, a blue tooth link, or others.

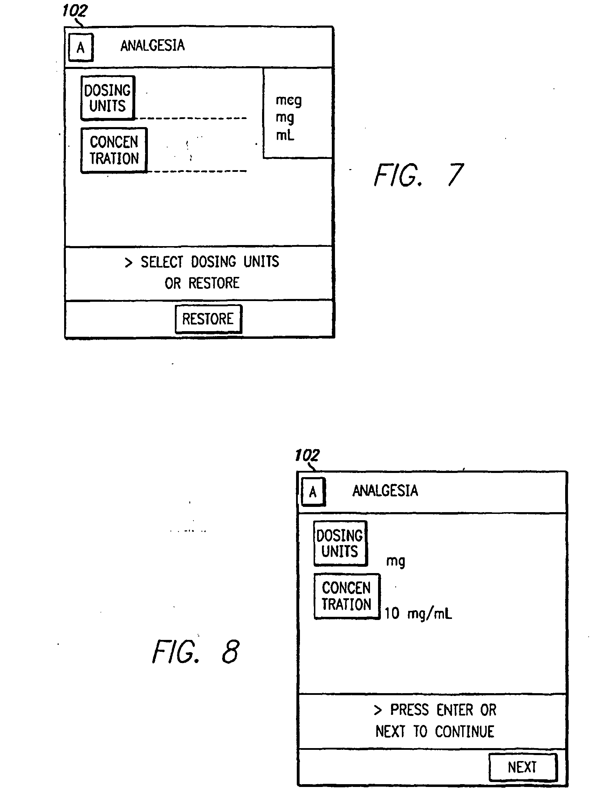

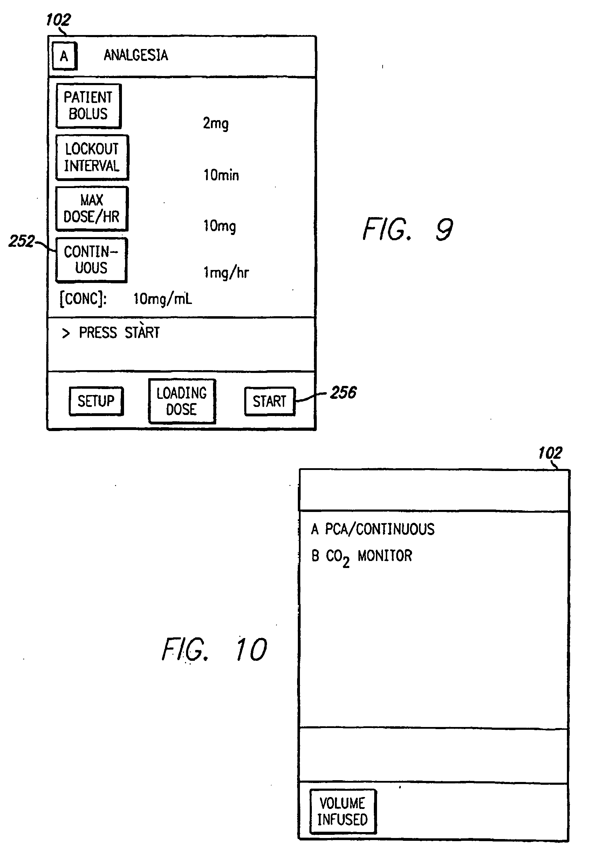

The clinician then selects the PCA unit 150A and its corresponding channel by depressing the SELECT key 156 on the PCA pump unit 150A (FIG. 1). By selecting the PCA pump unit 150A, the information display 102 is configured so as to act as the user interface and thus provides PCA pump function-specific displays and softkeys, as shown in FIGS. 7-9. In this example, the displays are PCA pump-specific. The clinician may first restore previous dosing units and the analgesic concentration or select the dosing units from, for example, mcg, mg, or ml, and input the analgesic concentration, as shown in FIGS. 7 and 8. Next, as shown in FIG. 9, the clinician may input or restore previous parameters for the patient bolus dosage. For additional precaution to further prevent respiratory and central nervous system depression and as an alternative embodiment of the present invention, the patient care system 90 or the pump unit 150A may require the clinician to enter the patient request dosing limits, such as maximum dose per hour or per 24-hour period.

After entering the patient bolus dosage parameters and/or other drug delivery parameters, the clinician may choose to administer a background continuous infusion of narcotic analgesics by pressing the softkey 106 adjacent the CONTINUOUS label 252. Use of a background infusion in combination with patient-requested doses provides a level of narcotic analgesia sufficient for periods of low activity such as when the patient is sleeping. Thus, when the patient wakes up and requires additional analgesia because of increased activity levels, the patient can self-administer additional narcotic analgesics to meet those needs. If a background continuous infusion is selected by pressing the softkey 106 adjacent the CONTINUOUS label 252, the information display 102 allows the clinician to input a desired continuous infusion dose. FIG. 9 shows the information display 102 after the clinician has entered values for both the patient bolus dose and the continuous dose.

For infusion parameters, such as the PCA infusion parameters shown in FIG. 9 (PATIENT BOLUS, LOCKOUT INTERVAL, MAX DOSE/HR, CONTINUOUS, and CONCENTRATION), a stored drug library may exist in the pump or patient care system that has preestablished values. These preestablished values may contain "hard" and "soft" limit values on dosing parameters and other infusion parameters. The limits may have been established by the clinic or institution within which the patient care system 90 resides. Once the values have been entered into the patient care system 90 by the clinician as shown in FIG. 9, the microprocessor controller 264, according to its programming will enter a verification stage in which it compares each of these selected values against the stored library to verify that the selected values are within acceptable ranges. If a selected value contravenes a hard limit, the microprocessor controller 264 may alarm and require a value change before operation of the patient care system 90 can begin. If the selected value contravenes a soft limit, the microprocessor controller 264 may require an acknowledgment from the clinician that he or she understands the value entered is outside a soft limit and that this value is nevertheless to remain in force. Although in the presently preferred embodiment, the drug library is stored in the patient care system, the library or libraries may be located elsewhere. For example, in the case where the patient care systems 90 is connected to a hospital server or other server, such a drug library may be located at the remote server and the patient care system 90 would communicate with the drug library stored in the remote server during the verification stage to obtain the acceptable ranges. As another example, the drug library may be located in a portable data assistant (herein "PDA") such as a Palm Pilot™, or in a portable computer such as a laptop computer, or in a patient bedside computer, or nurse's station computer, or other. Communications between the patient care system 90 and the remote drug library may be effected by infrared link, RF, blue tooth, or by other means. The clinician may carry the PDA having the drug library and before the patient care system 90 will begin operation, it must communicate with the PDA to compare the hard and soft limits against the entered values. Other library storage arrangements are possible.

Once the above steps have been completed, the clinician attaches the PCA administration set 254 (FIG. 2) to the patient's indwelling vascular access device (not shown) and presses the softkey 106 adjacent the START label 256 on the central interface unit 100. The pump unit 150A is now operating with continuous monitoring by the capnography unit 150B of the patient's expired CO₂ concentration and respiration rate. The PCA pump unit 150A begins background continuous infusion, if one has been selected. In addition, the patient may now request a dose of narcotic analgesics at any time by means of the patient dose request actuation device 135. Whether the patient actually receives a requested analgesic dose depends upon the patient request dosing limits, if any, as well as the patient's current ETCO₂ level and respiration rate relative to the limits set by the clinician.

Referring now to FIG. 10, the positions A and B in the information display 102 advise the clinician that the two functional units located at channel positions A and B are communicating with the central interface unit 100. The information display 102 may further be used to indicate the status of each functional unit occupying each respective channel in the patient care system 90. For example, the information display 102 at channel A, corresponding to the PCA unit 150A occupying channel A, can be configured to indicate the patient bolus dosage and the background continuous infusion dosage. In addition, the information display 102 at channel B, corresponding to the capnography unit 150B (also termed "CO₂ Monitor") occupying channel B, can be configured to indicate minimum and maximum ETCO₂ levels and respiration rates. The patient care system 90 may also be configured such that the information display 102 at channel B displays the patient's current percent ETCO₂ level and respiration rate. Naturally, if other monitors or pumps are attached, corresponding information from those units may also be displayed on the information display 102.

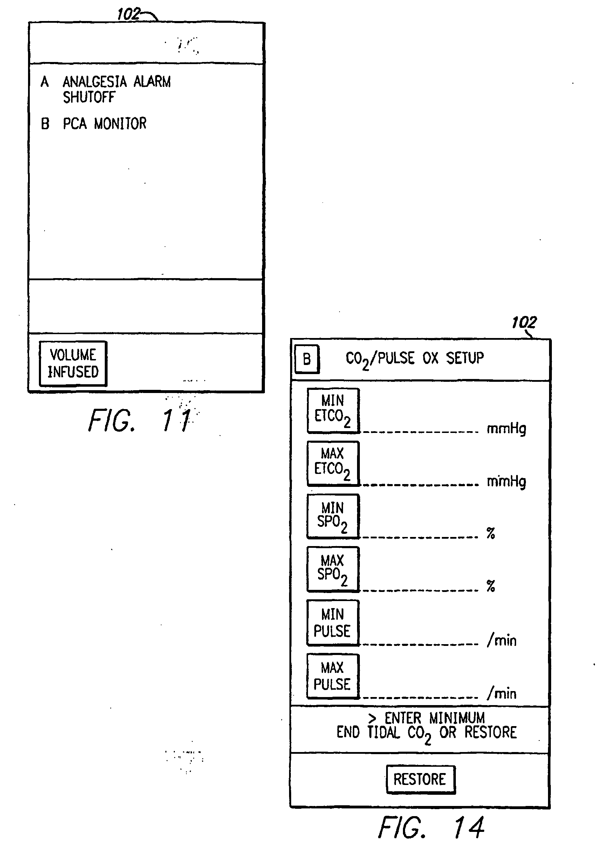

In the event that the patient's ETCO₂ value or respiration rate are outside the maximum and minimum levels set by the clinician, the central interface unit 100 immediately shuts-off the PCA pump unit 150A, and thereby stops further administration of any background infusion and bolus doses. Optionally, the patient care system 90 may be programmed to adjust, rather than stop, the background continuous flow rate or bolus dose in response to capnography data or data received from other attached monitors, if any. As illustrated in FIG. 11, position A of the information display 102 indicates ANALGESIA ALARM SHUTOFF status for the PCA pump unit 150A. In addition, the central interface unit 100 activates an audio alarm 276 through a speaker 278 or otherwise, displays a visual alarm on the information display 102, flashes the ALARM indicator 164 on the PCA pump unit 150A and/or capnography unit 150B, and sends an emergency signal via the interface ports 122 and the external communications controller 274 in order to alert appropriate medical personnel, such as by a nurse call. Thus, faster response and intervention by medical personnel of the patient's respiratory depression from the administration of narcotic analgesics is provided.

Referring now to FIG. 12, an alternative embodiment of a patient care system 300 in accordance with aspects of the present invention includes the interface unit 100, the pump unit 150A, and the capnography unit 150B as described above, and additionally includes a pulse oximetry unit 150C for providing the non-invasive measurement of blood oxygen saturation levels and pulse rate. The pulse oximetry unit 150C includes a pulse oximetry sensor 322, for example a dual wavelength sensor, that attaches to a portion of the patient containing venous flow, such as a finger 324 or earlobe. The pulse oximetry unit 150C receives signals from the sensor 322 through a connecting cable 326 and interprets the signals in accordance with the standard operation of a pulse oximeter as will be understood by persons of ordinary skill in the art. Examples of pulse oximetry sensors are disclosed in U.S. Pat. No. 5,437,275 to Amundsen et al. and U.S. Pat. No. 5,431,159 to Baker et al. From these sensor signals, the pulse oximetry unit 150C can determine the patient's percentage of blood oxygen saturation, the SpO₂, and the pulse rate. The pulse oximetry unit 150C contains an Sp0₂% display 310 to display the patient's percentage of oxygen saturation and a pulse display 320 to display the patient's pulse rate.

A user may program the patient care system 300, for example using program steps similar to those described with reference to FIGS. 5-10, to signal an alarm, display an advisory, shut off the pump unit 150A, or alter operation of the pump unit 150A if one or more of the ETCO₂, respiration rate, SpO₂, or pulse rate values, or some combination thereof, falls outside a selected range of acceptable values. In one embodiment, measurements from one or more of the functional modules 150B or 150C may initiate a program sequence in the interface unit 100 that terminates a particular fluid delivery protocol and initiates a new delivery protocol from the pump unit 150A or another attached pump module (not shown).

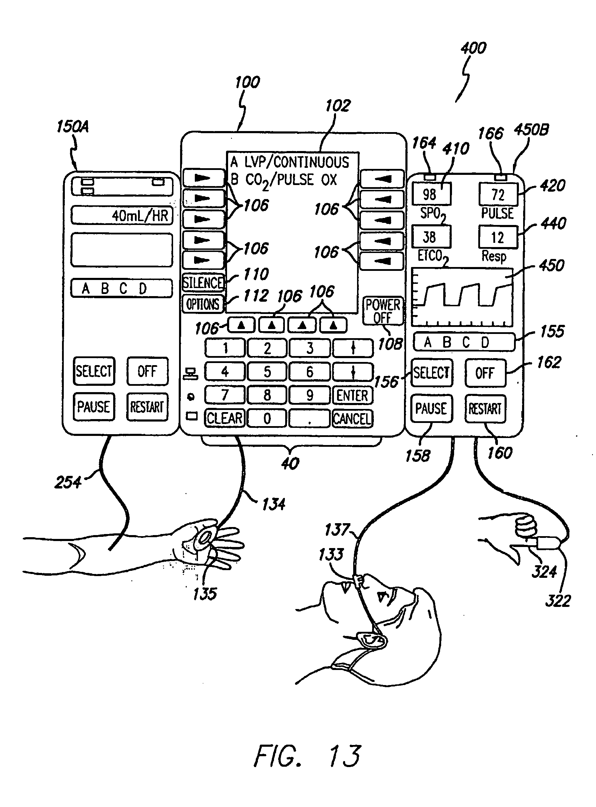

Referring to FIG. 13, another embodiment of a patient care system 400 incorporating aspects of the present invention includes an integrated capnography/pulse oximetry unit 450B. The capnography/pulse oximetry unit 450B combines the functions of the CO₂ unit 150B and the pulse oximetry unit 150C as described above into one integrated functional unit 450B. The capnography/pulse oximetry unit 450B includes displays for Sp0₂ 410, pulse 420, ETCO₂ 430, respiration rate 440, and the CO₂ waveform 442. The indicators 164, 166, and 155 and the switches 156, 158, 160, and 162 are as described above with respect to other embodiments. The integrated capnography/pulse oximetry unit 450B can be programmed by the user, or alternatively by program information stored in the memory 250 (FIG. 4) of the interface unit 100 or in the capnography/pulse oximetry unit 450B itself. FIG. 13 shows a PCA pump unit 150A connected at one side of an interface unit 100, and a combination CO₂ monitoring/pulse oximetry (SpO₂) unit 150B connected at the other side of the interface unit 100. Accordingly, the patient has in his hand a PCA dose request button 135 connected to the central interface unit 100 through a cable 134 for controlling a bolus of analgesic to be administered to himself from the PCA pump unit 150A through a fluid administration set 254. The patient is also monitored for his CO₂ level and respiration by a capnography unit forming a part of unit 150B. An expired air sampling device 133 is mounted in place at the patient's nose and mouth and communicates the expired air to the capnography part of unit 150B through the line 137. The patient is also monitored for blood oxygen saturation level with a pulse oximeter that forms a part of unit 150B. A pulse oximetry sensor 322 is connected to the patient's finger and the sensor signals are communicated to the pulse oximetry portion of the unit 150B through the cable 326.

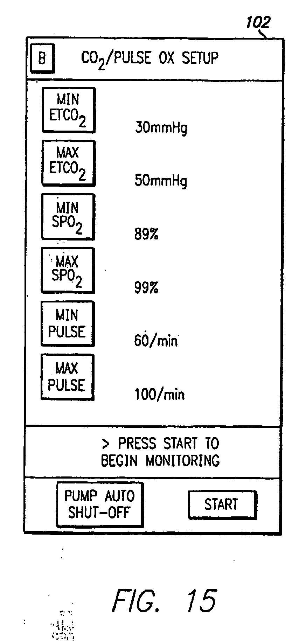

FIGS. 14 and 15 depict setup-screens displayed on the information display 102 directing the user to enter maximum and minimum values for each of the measured parameters and for initiating an infusion.

Referring to the block diagram of FIG. 16, an alternative embodiment of a patient care system 490 in accordance with aspects of the present invention comprises an integrated programmable infusion pump 500 with a pump drive unit 510, a user interface for entering 520 and displaying 530 information, a microprocessor controller 540 that controls and monitors the operation of the user interface 520, 530 and the pump drive unit 510, and a memory 550 in communication with the microprocessor controller 540 for storing program instructions for operating the patient care system 490 and may also store a library or libraries for drugs, pumping parameters, and physiological parameters usable with monitors. The infusion pump 500 is generally similar to the infusion pump disclosed in U.S. Pat. No. 5,800,387 by Duffy et al., which is incorporated herein by reference in its entirety. However, the patient care system 490 also includes a capnography unit 560 and a pulse oximeter unit 570 within the system housing 580. The microprocessor controller 540, like the central interface unit 100 of the above-described modular systems 10, 300, and 400, monitors values generated by the capnography unit 560 and/or the pulse oximeter unit 570 and affects operation of the pump drive unit 510 in response to pre-determined changes in the measured values.

Although various embodiments of the invention have been described and illustrated, the descriptions are intended to be merely illustrative. It will probably be apparent to those skilled in the art that modifications may be made to the embodiments as described without departing from the scope of the invention as set forth in the claims below. Accordingly, it is not intended that the invention be limited, except as by the appended claims.

### CONCEPTS

As short summaries, this writing has disclosed at least the following broad concepts.
Concept 1. A patient care system, comprising:
   a pump for delivery of a medical fluid to a patient;
   a controller in communication with the pump for controlling operation of the pump;
   a monitor unit that monitors the expiration air of the patient and provides a measured value of a selected component of the expiration air to the controller; and
   a memory with which the controller is connected, the memory comprising a stored range of acceptable values of the selected component of expiration air;
   wherein the controller compares the measured value of the selected component received from the monitor unit to the range of acceptable values for the component stored in the memory and if the measured value is outside the range stored in the memory, the controller performs a predetermined action.
Concept 2. The patient care system of concept 1 wherein the monitor unit monitors the expiration air of the patient for CO₂ and provides a measured value of the CO₂ to the controller.
Concept 3. The patient care system of concept 2 wherein the controller automatically adjusts the rate of delivery of the medical fluid in accordance with the CO₂ in the patient's expired air.
Concept 4. The patient care system of concept 3 wherein the controller automatically suspends delivery of the medical fluid by the pump to the patient if the measured value of the CO₂ in the expired air of the patient is outside the stored range of acceptable values.
Concept 5. The patient care system of concept 2 further comprising:
   a PCA dose request switch connected to the controller with which the patient may request the pump to infuse a quantity of analgesic;
   wherein prior to allowing the pump to infuse the quantity of analgesic, the controller compares the measured value of the C0₂ received from the monitor unit to the range of acceptable values for CO₂ stored in the memory and if the measured value is outside the range stored in the memory, the controller does not permit the pump to infuse the requested quantity of analgesic to the patient.
Concept 6. The patient care system of claim 2 further comprising:
   a PCA dose request switch connected to the controller with which the patient may request the pump to infuse a quantity of analgesic;
   wherein prior to allowing the pump to infuse the quantity of analgesic, the controller compares the rate of change of the CO₂ received from the monitor unit to the range of acceptable values for CO₂ stored in the memory and does not permit the pump to infuse the requested quantity of analgesic to the patient if the rate of change is not consistent with the acceptable values.
Concept 7. The patient care system of concept 2 further comprising a display on which is displayed a CO₂ waveform of the patient as derived from a series of measured CO₂ values provided by the monitor unit.
Concept 8. The patient care system of concept 1 wherein the monitor unit monitors the expiration air of the patient for end tidal C0₂ and provides a measured value of the end tidal CO₂ to the controller.
Concept 9. The patient care system of concept 8 wherein the controller automatically adjusts the rate of delivery of the medical fluid in accordance with the end tidal C0₂ in the patient's expired air.
Concept 10. The patient care system of concept 9 wherein the controller automatically suspends delivery of the medical fluid by the pump to the patient if the measured value of the end tidal C0₂ in the expired air of the patient is outside the stored range of acceptable values.
Concept 11. The patient care system of concept 1 wherein the memory in which the range of acceptable values of the selected component is stored is located at a position removed from the pump.
Concept 12. The patient care system of concept 1 wherein the memory in which the range of acceptable values of the selected component is stored is located in the pump.
Concept 13. The patient care system of concept 1 further comprising:
   an oximetry unit connected to the controller that monitors the blood of the patient and provides a measured value of the oxygen saturation of the patient's blood to the controller;
   wherein the memory comprises a stored range of acceptable values of the oxygen saturation of blood;
   wherein the controller compares the measured value of the oxygen saturation received from the oximetry unit to the range of acceptable values for the oxygen saturation stored in the memory and if the measured value is outside the range stored in the memory, the controller performs a predetermined action.
Concept 14. The patient care system of concept 13 wherein the controller automatically adjusts the rate of delivery of the medical fluid in accordance with either of the C0₂ in the patient's expired air or the oxygen saturation of the patient's blood.
Concept 15. The patient care system of concept 13 wherein the oximetry unit also monitors the pulse rate of the patient and provides a measured value of the pulse rate to the controller;
   wherein the memory comprises a stored range of acceptable values of the pulse rate;
   wherein the controller compares the measured value of the pulse rate received from the oximetry unit to the range of acceptable values for the pulse rate stored in the memory and if the measured value is outside the range stored in the memory, the controller performs a predetermined action.
Concept 16. The patient care system of concept 15 wherein the controller automatically adjusts the rate of delivery of the medical fluid in accordance with any of the C0₂ in the patient's expired air, the oxygen saturation of the patient's blood, or the patient's pulse rate.
Concept 17. A patient care system, comprising:
   a pump for intravenous delivery of a drug to a patient;
   a microprocessor controller in communication with the pump for controlling operation of the pump; and
   a capnography unit for monitoring the concentration of carbon dioxide in the patient's expired air, said capnography unit communicating with the microprocessor controller and cooperating therewith to cause a change in the delivery of the drug to the patient in response to the concentration of carbon dioxide exceeding a predetermined levels as measured by said monitor.
Concept 18. The patient care system of concept 17 wherein the change is an automatic suspension of the delivery of the drug.
Concept 19. The patient care system of concept 17 wherein the change comprises a modulation in a rate of delivery of the drug, wherein the microprocessor controller adjusts the rate of delivery in accordance with the concentration of carbon dioxide in the patient's expired air.
Concept 20. The patient care system of concept 17 wherein the capnography unit and the microprocessor controller cooperate to cause a change in the delivery of the drug to the patient in response to the end tidal carbon dioxide concentration as measured by said monitor being above a maximum predetermined level or below a minimum predetermined level.
Concept 21. The patient care system of concept 20 further comprising a pulse oximeter in communication with the microprocessor controller, wherein the pulse oximeter measures oxygen saturation level of the patient's blood and pulse rate, and the microprocessor changes the rate of delivery of the drug if any of the carbon dioxide concentration, the oxygen saturation, or the pulse rate fall outside the predetermined levels.
Concept 22. The patient care system of concept 21 wherein the microprocessor calculates an index from the end tidal carbon dioxide concentration, the oxygen saturation, and wherein the microprocessor changes the rate of delivery of the drug if the calculated value falls outside of a predetermined index range.
Concept 23. A patient care system, comprising:
   a pump capable of intravenous delivery of a drug to a patient;
   control means for permitting the patient to self-administer analgesia using said pump; and
   a capnography monitor monitoring the concentration of carbon dioxide in the patient's expired air, said capnography monitor cooperating with said delivery of analgesia to automatically prevent administration of analgesia using the patient control means in response to the concentration of carbon dioxide falling outside predetermined levels as measured by said monitor.
Concept 24. The system according to concept 23 wherein said capnography monitor further monitors respiration rate; and said monitor cooperates with the pump to automatically prevent administration of analgesia using the patient control means when the respiration rate falls outside predetermined levels.
Concept 25. The system according to concept 24 wherein said system further comprises a pulse oximeter in communication with the pump.
Concept 26. The system according to concept 23 further comprising interface means between said pump and said capnography monitor, said interface means including a processor means providing communication between the pump and capnography monitor and providing said cooperation between said delivery of analgesia and said monitor.
Concept 27. A method for controlling patient self-administration of fluid infusions comprising:
   monitoring patient conditions by connecting a capnography unit to the patient, wherein the capnography unit is capable of monitoring the concentration of carbon dioxide in the expired air of a patient;
   connecting the capnography unit to an interface unit including a microprocessor and a user interface adapted to provide an interface with a user;
   inputting patient condition limits into the interface unit;
   comparing monitored patient conditions to patient condition limits in said interface and generating a signal indicative of said comparison;
   connecting the patient to an infusion unit, wherein the infusion unit communicates with the interface unit, said infusion unit is capable of performing fluid infusion to the patient in accordance with infusion unit specific information;
   requesting fluid infusions from the infusion unit by patient activation of the infusion unit;
   performing fluid infusion with the infusion unit in accordance with a predetermined infusion protocol in response to said patient activation and in response to a signal from the interface unit representative of the monitored patient conditions being within said patient condition limits; and
   disabling the infusion unit and terminating the fluid infusion in response to a signal from the interface unit representative of the monitored patient conditions being outside the patient condition limits.
Concept 28. The method of concept 27 wherein the monitoring step comprises monitoring the patient's end tidal C0₂ concentration and respiration rate, and wherein the patient condition limits are minimum C0₂ level, saturation level, and minimum respiration rate.
Concept 29. The method of concept 28 wherein the monitoring step further comprises monitoring the patient's blood oxygen saturation level and pulse rate using a pulse oximeter associated with the interface unit, and wherein the patient condition limits comprise a blood oxygen saturation level and a pulse rate.
Concept 30. The method of concept 27 wherein the predetermined infusion protocol for the infusion unit comprises bolus dose delivery parameters, including bolus dose, duration of bolus dose infusion, and rate of bolus dose infusion.
Concept 31. The method of concept 27 wherein the predetermined infusion protocol for the infusion unit further comprises one or more patient request dosage limits, selected from the group comprising minimum interval of time between doses, maximum total number of doses, maximum number of bolus doses over a predetermined period of time, and maximum total volume of patient therapy to be infused.
Concept 32. The method of concept 27 further comprising the step of providing a continuous infusion by the infusion unit.
Concept 33. A drug infusion pump for use with a container containing a particular drug, said pump comprising:
   a pump mechanism that causes the particular drug to be delivered to a patient from the container;
   a programmable controller controlling the pump mechanism;
   a monitor unit that monitors the expiration air of a patient to measure a selected component of that air, and that provides a measured value representative of the measured component;
   a memory storing a drug library, the drug library containing a plurality of drug entries, there being associated with each drug entry a set of associated drug delivery parameters for configuring the drug infusion pump, the memory also storing the selected component of the patient's expiration air, there being associated with the selected component a range of acceptable values;
   a user interface enabling a user to program the programmable controller, said user interface comprising:
      means for enabling the user to select a drug entry from the electronically loaded drug library; and
      means for configuring the programmable controller with the set of drug delivery parameters associated with the selected drug entry;
      wherein the programmable controller is configured to receive the measured value, compare the measured value to the range of acceptable values of the selected component, and to control the pump mechanism in accordance with the comparison.
Concept 34. The drug infusion pump of concept 33 wherein the memory is electronically loadable.
Concept 35. The drug infusion pump of concept 34 further comprising input circuitry through which the electronically loadable memory is electronically loaded with the drug library and with the selected component of the patient's expiration air with a range of acceptable values for the selected component.
Concept 36. The drug infusion pump of claim 33 wherein:
   the memory also stores limit values for ETC0₂ ;
   the monitor unit monitors the expiration air of a patient to measure ETC0₂ and provides a measured value representative of the measured ETC0₂; and
   the programmable controller is configured to receive the measured value of the ETC0₂, compare the measured ETC0₂ to the range of acceptable ETC0₂ values from the memory, and to control the pump mechanism in accordance with the comparison.
Concept 37. The drug infusion pump of concept 36 wherein:
   the memory also stores limit values for respiration;
   the monitor unit monitors the expiration air of a patient to measure the respiration and provides a measured value representative of the measured respiration; and
   the programmable controller is configured to receive the measured value of the respiration, compare the measured respiration value to the range of acceptable respiration values from the memory, and to control the pump mechanism in accordance with the comparison.
Concept 38. The drug infusion pump of concept 33 wherein:
   associated with drug entries in the drug library is a set of hard limit values that have been established by an institution within which the drug infusion pump resides;
   the controller being programmed to compare pumping parameters entered by an operator against the stored hard limits in the drug library, and if an entered pumping parameter contravenes a hard limit, the controller provides an alarm and requires a change in the pumping parameter before operation of the pump can begin.
Concept 39. The drug infusion pump of concept 33 wherein:
   associated with drug entries in the drug library is a set of soft limit values that have been established by an institution within which the drug infusion pump resides;
   the controller being programmed to compare pumping parameters entered by an operator against the stored soft limits in the drug library, and if an entered pumping parameter contravenes a soft limit, the controller requires an acknowledgment from a user that the limit value has been contravened and that this value is nevertheless to remain in force.
Concept 40. The drug infusion pump of concept 33 wherein the memory is located inside the pump.
Concept 41. The drug infusion pump of concept 33 wherein the memory is located remotely from the pump mechanism.
Concept 42. The drug infusion pump of concept 33 wherein the memory is located in a portable unit that may be carried on the person of a healthcare provider.
Concept 43. A drug infusion pump for use with a container containing a given drug, said container including a machine readable label, the label specifying an identifier of the given drug and possibly other information about the given drug, said pump comprising:
   a pump mechanism which during operation causes the given drug to be delivered to a patient from the container;
   a programmable controller controlling the pump mechanism;
   a monitor unit that monitors the expiration air of a patient to measure a selected component of that air, and that provides a measured value representative of the measured component;
   a memory storing a drug library, said drug library containing a plurality of drug entries, there being associated with each drug entry a set of associated drug delivery parameters for configuring the drug infusion pump, the memory also storing the selected component of the patient's expiration air, there being associated with the selected component a range of acceptable values;
   a label reader which during use reads the contents of the label on the container; and
   means responsive to the label reader for identifying an entry in the drug library that corresponds to the given drug and configuring the programmable controller by using the set of drug delivery parameters associated with the identified entry from the drug library;
   wherein the programmable controller is configured to receive the measured value, compare the measured value to the range of acceptable values of the selected component, and to control the pump mechanism in accordance with the comparison.

## Claims

1. A system for monitoring medication delivery to a patient while the patient is in a healthcare facility, the monitoring system comprising:
a memory in which is stored identification data about the patient, medication delivery data about the patient, data for infusions in progress, and vital signs data about the patient;
a display on which identification data, medication delivery data, and vital signs data about the patient is displayed; and
a processor coupled to the memory and the display, the processor configured to receive patient identification data, medication delivery data, and patient vital signs data about the patient, including infusion delivery data for infusions in progress, from multiple sources in the healthcare facility while the patient is in the healthcare facility and store the identification data and medication delivery data in the memory; and wherein the processor is further configured to retrieve the patient identification data, the medication delivery data, the delivery data for infusions in progress, and the vital signs data from the memory in response to a request therefor, format the retrieved data in accordance with a format request, and display the retrieved and formatted data on the display.

2. The system of claim 1 wherein the monitoring system further comprises an infusion pump.

3. The system of claim 1 wherein one of the multiple data sources from which the processor receives medication delivery data is a portable data device.

4. The system of claim 3 wherein the processor is further configured to receive medication delivery data from the portable data device through a wireless connection.

5. The system of claim 3 wherein the portable data device is configured to receive medication delivery data from an infusion pump and transfer that medication delivery data to the processor.

6. The system of claim 3 wherein the processor also is further configured to retrieve patient identification data and medication delivery data from the memory and transmit the retrieved data to the portable data device.

7. The system of claim 1 wherein one of the multiple data sources from which the processor receives medication delivery data is a server.

8. The system of claim 1 wherein the processor is further configured to format retrieved medication delivery data to display medication delivered to the patient organized by time.

9. The system of claim 1 wherein the processor is further configured to format retrieved medication delivery data to display medication delivered to the patient organized by unit of measure.
